(19) Europäisches Patentamt
European Patent Office
Office européen des brevets

(11) EP 0 868 900 A1

(12) EUROPÄISCHE PATENTANMELDUNG

(43) Veröffentlichungstag:
**07.10.1998 Patentblatt 1998/41**

(51) Int. Cl.$^6$: **A61K 7/09**

(21) Anmeldenummer: **98102871.5**

(22) Anmeldetag: **19.02.1998**

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(30) Priorität: **14.03.1997 DE 19710544**

(71) Anmelder:
**Wella Aktiengesellschaft**
**64274 Darmstadt (DE)**

(72) Erfinder:
- **Lang, Günther, Dr.**
  **64354 Reinheim (DE)**
- **Walther, Heiko, Dr.**
  **35037 Marburg/Lahn (DE)**
- **Dannecker, Beate, Dr.**
  **64283 Darmstadt (DE)**
- **Hanefeld, Wolfgang, Prof. Dr.**
  **35037 Marburg/Lahn (DE)**

(54) **Mittel und Verfahren zur daurerhaften Haarverformung**

(57) Mittel und Verfahren zur dauerhaften Haarverformung, unter Verwendung von Mercaptoethylaminen oder ihren physiologisch verträglichen Salze der Formel

$$HS - CH_2 - CH_2 - NR_1 R_2 \quad (I)$$

in der $R_1$ Wasserstoff, ein geradkettiger oder verzweigter Alkyl-, Hydroxyalkyl- oder Polyhydroxyalkylrest mit 1 bis 6 Kohlenstoffatomen bedeutet;
$R_2$ ein geradkettiger oder verzweigter Hydroxyalkyl- oder Polyhydroxyalkylrest mit 1 bis 6 Kohlenstoffatomen, Cyclohexyl oder Furfuryl sein kann; oder
$R_1$ und $R_2$ gemeinsam mit dem Stickstoffatom einen fünf- oder sechsgliedrigen Ring bilden, der noch ein anderes Heteroatom enthalten kann und mit einer Hydroxyl- oder Carboxylgruppe oder mit einem oder zwei 1 bis 4 Kohlenstoffatome umfassenden Alkyl-, Hydroxyalkyl-, Polyhydroxyalkylrest oder Alkoxycarbonylrest substituiert sein kann.

EP 0 868 900 A1

## Beschreibung

Die vorliegende Erfindung betrifft sowie Mittel zur dauerhaften Haarverformung, welche als keratinreduzierenden Wirkstoff Mercaptoethylamine und ihre Salze enthalten sowie ein Verfahren zur dauerhaften Haarverformung unter Verwendung derartiger Mittel.

Bekanntlich beruht die klassische Technik zur Durchführung einer dauerhaften Haarverformung auf zwei Behandlungsschritten: Im ersten Schritt werden die Cystin-Disulfid-Brücken des Haarkeratins durch Einwirken eines Mittels, welches einen reduzierenden Wirkstoff enthält (Verformungsmittel), geöffnet. Sodann wird das Haar in die gewünschte Form gebracht. In einem zweiten Schritt werden Cystin-Disulfid-Bindungen unter Verwendung eines Fixiermittels, d.h. eines einen oxidierenden Wirkstoff enthaltenden Mittels, wieder geschlossen.

Als klassisches Dauerwellreduktionsmittel wird, wie die Pionierarbeiten in den deutschen Patentschriften 948 186 und 972 424 zeigen, die Thioglykolsäure, zum Beispiel als Ammonium- oder Monoethanolaminsalz, verwendet. Weitere übliche reduzierende Wirkstoffe sind anorganische Sulfite, 2-Mercapto-propionsäure (Thiomilchsäure), 3-Mercapto-propionsäure, bestimmte Mercaptocarbonsäureester, Cystein und Derivate dieser Verbindungen.

Alle diese Mittel weisen jedoch eine Reihe von Nachteilen auf. Alkalisch eingestellte Zubereitungen auf Basis der Mercaptocarbonsäuren zeigen trotz ausreichender Wirkung eine Haarschädigung, die sich beispielsweise in vermehrt auftretendem Haarbruch äußert. Vielfach belasten diese Mittel auch in unerwünschter Weise die Kopfhaut.

Schließlich erfordert der unangenehme Geruch der verwendeten Reduktionsmittel eine intensive Parfümierung der Produkte. Durch Verwendung von 2-Mercaptopropionsäure (Thiomilchsäure) ist man in der Lage, einige der erwähnten Probleme zu lösen. Allerdings zeichnet sich die Thiomilchsäure im Vergleich zur allgemein verwendeten Thioglykolsäure durch eine schwächere Umformung aus.

Die Mercaptocarbonsäureester, welche eine Haarverformung auch bei niedrigeren pH-Werten ermöglichen, sind bezüglich ihrer Hautverträglichkeit sowie ihres Sensibilisierungsrisikos nicht zufriedenstellend. Anstelle der Mercaptocarbonsäureester wurden auch Mercaptocarbonsäureamide wie Thioglykolsäureamid oder alkyl- bzw. hydroxyalkylsubstituierte Amide verwendet. Derartige Verbindungen sind aus den Patentschriften WO-A-91/10421 und EP-A-0 455 457 bekannt. Diese Stoffe haben, wie die Carbonsäureester, ein hohes Umformungspotential auch bei niedrigen pH-Werten, sind jedoch in Bezug auf die Sensibilisierung noch kritischer als die Ester. Mercaptoethylamine und ihre Salze, in denen das Stickstoffatom mit zwei gleichen geradkettigen oder verzweigten Alkylresten mit 1 bis 6 Kohlenstoffatomen substituiert ist, sind bereits aus der deutschen Patentanmeldung 195 03 131 bekannt.

Es wurde nun überraschend gefunden, daß sich die genannten Nachteile durch die Verwendung der nachfolgend beschriebenen Mercaptoethylamine vermeiden lassen, und daß diese über ein stärkeres Umformungspotential als Thiomilchsäure verfügen.

Es sind dies Mercaptoethylamine und ihre physiologisch verträglichen Salze der Formel

$$HS - CH_2 - CH_2 - NR_1 R_2 \qquad (I),$$

in der $R_1$ Wasserstoff, einen geradkettigen oder verzweigten Alkyl-, Hydroxyalkyl- oder Polyhydroxyalkylrest mit 1 bis 6 Kohlenstoffatomen bedeutet;

$R_2$ ein geradkettiger oder verzweigter Hydroxyalkyl- oder Polyhydroxyalkylrest mit 1 bis 6 Kohlenstoffatomen, Cyclohexyl oder Furfuryl ist; oder

$R_1$ und $R_2$ gemeinsam mit dem Stickstoffatom einen fünf- oder sechsgliedrigen Ring bilden, der noch ein anderes Heteroatom enthalten kann und mit einer Hydroxyl- oder Carboxylgruppe oder mit einem oder zwei 1 bis 4 Kohlenstoffatome umfassenden Alkyl-, Hydroxyalkyl-, Polyhydroxyalkyl- oder Alkoxycarbonylrest substituiert sein kann.

Die Herstellung der Mercaptoethylamine der Formel (I) erfolgt durch Umsetzung der entsprechenden Amine mit Thiiran in Toluol bei 90 bis 100°C und anschließende fraktionierte Destillation.

Gegenstand der vorliegenden Erfindung ist somit ein Mittel zur dauerhaften Verformung der Haare, das als keratinreduzierenden Wirkstoff ein Mercaptoethylamin der Formel (I) oder eines seiner physiologisch verträglichen Salze, z.B. das Hydrochlorid, enthält.

Besonders bevorzugt sind Mittel zur dauerhaften Verformung der Haare, welche Mercaptoethylamine der Formeln

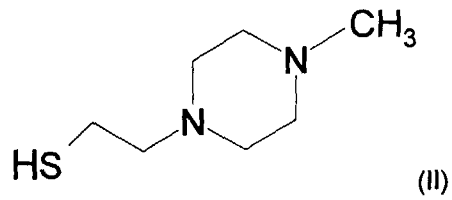 (II) oder 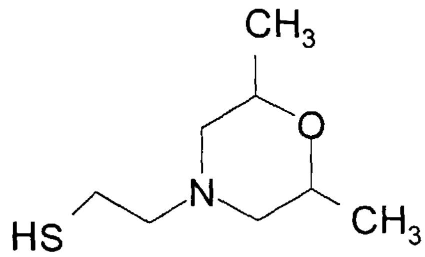 (III)

enthalten.

Die Mercaptoethylamine werden einem derartigen Mittel zur dauerhaften Haarverformung in eine Menge von 3 bis 28 Gewichtsprozent, vorzugsweise 5 bis 21 Gewichtsprozent, eingesetzt. Sie können auch im Gemisch mit anderen bekannten Thiolen wie Thioglykolsäure, Thiomilchsäure, Cystein, Cysteamin und Alkyl- oder Acylcysteamin oder Sulfiten eingesetzt werden.

Die gebrauchsfertigen Haarverformungsmittel besitzen bevorzugt einen pH-Wert von 6,5 bis 9,5, besonders bevorzugt von 6,5 bis 8,5. Als Alkalisierungsmittel bzw. als Mittel zur Einstellung des pH-Wertes kommen insbesondere Ammoniak oder Natronlauge, aber auch wasserlösliche, physiologisch verträgliche Salze von organischen und anorganischen Basen, wie zum Beispiel Ammoniumhydrogencarbonat, in Betracht.

Das Verformungsmittel kann sowohl ein- als auch zweikomponentig verpackt angeboten werden, wobei das Mittel in Form einer wäßrigen Lösung oder einer Emulsion als auch in verdickter Form auf wäßriger Basis, insbesondere als Creme, Gel, Schaum oder Paste vorliegen kann.

Selbstverständlich kann das Verformungsmittel alle für derartige Mittel üblichen und bekannten Zusatzstoffe, zum Beispiel Verdickungsmittel, wie beispielsweise Bentonit, Fettsäuren, Stärke, Polyacrylsäure und deren Derivate, Cellulosederivate, Alginate, Vaseline, Paraffinöle; Netzmittel oder Emulgatoren aus den Klassen der anionischen, kationischen, amphoteren oder nichtionogenen oberflächenaktiven Substanzen, beispielsweise Fettalkoholsulfate, Fettalkohol-ethersulfate, Alkylsulfonate, Alkylbenzolsulfate, quaternäre Ammoniumsalze, Alkylbetaine, oxethylierte Alkylphenole, Fettsäurealkanolamide oder ethoxylierte Fettsäureester; ferner Trübungsmittel, wie zum Beispiel Polyethylenglykolester, Alkohole, wie zum Beispiel Ethanol, Propanol, Isopropanol und Glycerin; Zucker wie zum Beispiel D-Glucose, Lösungsvermittler, Stabilisatoren, Puffersubstanzen, Parfümöle, Farbstoffe sowie haarkonditionierende und haarpflegende Bestandteile, wie zum Beispiel kationische Polymere, Lanolinderivate, Cholesterin, Pantothensäure und Betain enthalten.

Die erwähnten Bestandteile werden in den für solche Zwecke üblichen Mengen verwendet, zum Beispiel die Netzmittel und Emulgatoren in Konzentrationen von insgesamt 0,2 bis 30 Gewichtsprozent, die Alkohole in einer Menge von insgesamt 0,1 bis 20 Gewichtsprozent, die Trübungsmittel, Parfümöle und Farbstoffe in einer Menge von jeweils 0,01 bis 1 Gewichtsprozent, die Puffersubstanzen in einer Menge von insgesamt 0,1 bis 10 Gewichtsprozent, Zucker, Stabilisatoren, sowie haarkonditionierende und haarpflegende Bestandteile in einer Menge von jeweils 0,1 bis 5 Gewichtsprozent, während die Verdickungsmittel und Lösungsvermittler in einer Menge von insgesamt 0,5 bis 20 Gewichtsprozent in diesem Mittel enthalten sein können.

Weiterhin können diesem Mittel zur Wirkungssteigerung sog. Quell- und Penetrationsstoffe, wie zum Beispiel Dipropylenglykolmonomethylether, 2-Pyrrolidon oder Imidazolidin-2-on, in einer Menge von 1 bis 30 Gewichtsprozent sowie zur Vermeidung einer Überkrausung der Haare Dithioverbindungen, beispielsweise Dithiodiglykolsäure, Dithiomilchsaure, die Disulfide der genannten Verbindungen oder die jeweiligen Salze, zugesetzt werden.

Durch Variation des pH-Wertes kann ein Mittel zur Verfügung gestellt werden, das universal für jede Haarstruktur, ggf. unter zusätzlicher Wärmeeinwirkung, geeignet ist. Das Mittel bewirkt eine elastische, dauerhafte und gleichmäßige Umformung vom Haaransatz bis zur Haarspitze, ohne allergische oder sensibilisierende Reaktionen hervorzurufen.

Die vorliegende Erfindung betrifft weiterhin ein Verfahren zur dauerhaften Haarverformung, bei dem man die Haare bevor und/oder nachdem man sie in die gewünschte Form bringt, mit einem Verformungsmittel behandelt, mit Wasser spült, sodann oxidativ behandelt, mit Wasser spült, ggf. zur Wasserwelle legt und sodann trocknet, welches dadurch gekennzeichnet ist, daß als Verformungsmittel die vorstehend beschriebenen erfindungsgemäßen Mittel verwendet werden.

Bei einer bevorzugten Ausführungsform des erfindungsgemäßen Verfahrens wird das Haar zunächst mit einem Shampoo gewaschen und danach mit Wasser gespült. Anschließend wird das handtuchtrockene Haar in einzelne Strähnen aufgeteilt und auf Wickler mit einem Durchmesser von 5 bis 30 mm, bevorzugt 5 bis 15 mm, gewickelt.

Sodann wird das Haar mit einer für die Haarverformung ausreichenden Menge, vorzugsweise 60 bis 120 g, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 g verwendet.

Für die oxidative Nachbehandlung im aufgewickelten oder abgewickelten Zustand kann jedes beliebige, für eine derartige Behandlung geeignete Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationische Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen. Diese üblichen Zusätze können insbesondere in einer Menge von 0,1 bis 10 Gewichtsprozent in dem Nachbehandlungsmittel enthalten sein.

Anschließend werden die Wickler entfernt. Falls erforderlich, kann das abgewickelte Haar nun nochmals oxidativ nachbehandelt werden. Sodann wird das Haar mit Wasser gespült, ggf. zur Wasserwelle gelegt und schließlich getrocknet.

Die nachfolgenden Beispiele sollen den Gegenstand der Erfindung näher erläutern, ohne jedoch den Gegenstand auf diese Beispiele zu beschränken.

**Beispiel 1: Herstellung Von 2,6-Dimethy-4-(2'-mercapto-ethyl)-morpholin**

In einem 500 ml Dreihalskolben werden 115,2 g (1 mol) 2,6-Dimethylmorpholin in 250 ml Toluol auf 90°C erhitzt. Dann tropft man 60,12 g (1 mol) Thiiran zu und erhitzt 5 Stunden lang unter Rückfluß. Der Ansatz wird am Umlaufverdampfer im Vakuum eingeengt und über eine Vigreux-Kolonne fraktioniert destilliert.

Die Ausbeute beträgt 143,5 g der Titelverbindung (82%).

**Analytik:**

a) $^{1}$H-NMR ($CDCl_3$):

α (ppm)= 3,99 + 3,64 (2x m, 2H, CH)
2,67 - 2,5 (m,6H, HS-$CH_2$-$\underline{CH_2}$-N + 2x N-$CH_2$)
1,74 (t, 2H, HS-$\underline{CH_2}$-$CH_2$-N)
1,21-1,1 (2xd,6H,$CH_3$)

b) $^{13}$C-NMR ($DCDl_3$):

α (ppm)= 71,59 (2xCH)
60,78 (HS-$CH_2$-$\underline{CH_2}$-N)
59,01 (2 C-Atome, N-$\underline{CH_2}$-$\underline{CH}$-O)
21,68 (HS-$CH_2$)
19,06 (2 C-Atome, $CH_3$)

c) MS (70 e.V. EI, RT)

m/z (%)= ($M^+$)= 175 (1,3),
129 (9,0),
128 (100),
98 (2.6),
70 (9,41),
43 (19,11),
42 (27,60)

d) Thioltitration: 95,48%

e) Elementaranalyse: $C_9H_{19}NOS$ (MG: 175,26)

Ber.: C: 54,82,
H: 9,77,
N: 7,99,
S: 18,29

Gef.: C: 55,15,
H: 9,27,
N: 8,10,
S: 18,01

f) IR NaCL-Platten): 2972-2810s ($CH_2$)
2546w (SH)

g) HPLC: Die HPLC ergab ein Ergebnis von 94,23 Flächenprozent für die Verbindung. (Säule: C 18 5U, 250 mm x 4,6 mm; Fließmittel Acetonitril: Puffer [4g $KH_2PO4$ +0,8g Octansulfonsäure-Na-Salz + 2 ml $H_3PO_4$] = 25:75; Flußrate 0,5 ml/min; Wellenlänge 200 mm)

h) pKs: 6,559 ($H_2O$)

i) UV-max: <200 mm (Acetonitril: Puffer = 25:75)

j) Siedepunkt: 55°C/0,05 Torr

**Beispiel 2: Herstellung Von 2,6-Dimethyl-1-(2'-mercapto-ethyl)-piperidin**

In einem 500 ml Dreihalskolben werden 113,2 g (1 mol) 2,6-Dimethylpiperidin in 250 ml Toluol auf 90°C erhitzt. Dann tropft man 60,12 g (1 mol) Thiiran zu und erhitzt 5 Stunden unter Rückfluß. Der Ansatz wird am Umlaufverdampfer im Vakuum eingeengt und über eine Vigreux-Kolonne fraktioniert destilliert.

Die Ausbeute beträgt 60,55 g der Titelverbindung (35%).

**Analytik:**

a) $^1$H-NMR ($CDCl_3$):

$\alpha$ (ppm)= 2,87 (m, 2H, HS-$CH_2$-$\underline{CH_2}$-N)
2,53 (m, 2H, HS-$\underline{CH_2}$-$CH_2$-N)
2,49 (m, 2H, N(-$\underline{CH}$-$CH_2$-)$_2$)
1,54 (t, 1H,HS)
1,53 (m, 2H, N-CH-$CH_2$-$\underline{CH_2}$)
1,23 (m, 2H, N(-CH-$\underline{CH_2}$-)$_2$)
1,08 (d, 6H, $CH_3$)

b) $^{13}$C-NMR ($DCDl_3$):

$\alpha$ (ppm)= 55,90 (2xCH)
52,53 (S-$CH_2$-$\underline{CH_2}$-N)
34,62 (2 C-Atome, H-CH-$\underline{CH_2}$-)
24,55 (HS-$CH_2$)
21,36 (2 C-Atome, $CH_3$)
20,15 (H-CH-$CH_2$-$\underline{CH_2}$-)

c) MS (70 e V, EI, 60°C)

m/z (%)= ($M^+$)= 173 (0,8),

172 (4,4),
126 (100,
98 (11,21),
70 (4,58),
56 (5,6),
42 (7,43)

d) Thioltitration: 99,66%

e) Elementaranalyse: $C_9H_{19}NS$ (MG: 173,26)

| | |
|---|---|
| Ber.: | C: 62,39, |
| | H: 11,05, |
| | N: 8,09, |
| | S: 18,50 |
| Gef.: | C: 62,40, |
| | H: 11,22, |
| | N: 8,00, |
| | S: 18,22 |

f) IR (NaCl-Platten): 2966-2788s ($CH_2$)
2591w (SH)

g) HPLC: Die HPLC ergab ein Ergebnis von 100 Flächenprozent für die Verbindung. (Säule: C 18 5U, 250 mm x 4,6 mm; Fließmittel: Puffer [4 g $KH_2PO4$ + 0,8 g Octansulfonsäure-Na-Salz + 2m $H_3PO_4$] = 25:75; Flußrate 0,5 ml/min; Wellenlänge 200 mm)

h) pKs: 7,72 ($H_2O$)

i) UV-max: <200 mm (Acetonitril: Puffer = 25:75)

j) Siedepunkt: 51°C/0,1 Torr

**Beispiel 3: Vergleich der Wellwirksamkeit**

Die Wellwirksamkeit der 2-Mercaptoethylamine, gemessen als normierte Wellstabilität (WSN), wurde unter Verwendung von Glycerinmonothioglykolat als Vergleichssubstanz mit Hilfe von Wellösungen bei pH = 7,8 und 9 bestimmt. Hierzu wurden 16,5 cm lange, vorgebleichte und damit geschädigte Zählhaarsträhnen (bestehend aus ca. 100 Haaren) aus mitteleuropäischem Haar naß auf genormte Spiralwickler (Innendurchmesser: 3 mm) aufgewickelt und nach dem Konditionieren im Klimaraum (Temperatur: 20°C; Luftfeuchte: 65%) mit einer 87 mmol/lOO g enthaltenden, auf den jeweiligen pH-Wert eingestellten Lösung der Reduktionsmittel behandelt. Die Auftragsmenge an Wellflüssigkeit wurde über das Verhältnis 1:1,2 errechnet (l g Haar - 1,2 ml Wellflüssigkeit). Als Einwirkzeit wurden 20 Minuten gewählt; die Einwirktemperatur betrug 50°C. Anschließend wurden die Haare mit einer peroxidhaltigen Fixierung fixiert, getrocknet und nach dem Abwickeln vier Stunden in Wasser (Wasserbadtemperatur: 40°C) ausgehängt.

Die Wellstabilität errechnet sich gemäß folgender Formel:

$$\text{Wellstabilität in \%} = \frac{l_o - l_t}{l_o - l_1} \times 100$$

$l_o$ = Gesamtlänge der nicht umgeformten, gestreckten Strähne(16,5 cm)

$l_t$ = Länge der abgewickelten, ausgehängten Strähne nach 240 Minuten

$l_1$ = Länge der umgeformten, aufgewickeiten Strähne bei einem Wickelinnendurchmesser von 3 mm beträgt diese: $l_1$ = 35 mm

Als Standard wurden Strähnchen mit einer entsprechend auf pH 9 eingestellten Glycerinmonothioglykolat-Lösung behandelt. Die nachfolgend in Tabelle 1 angegebenen normierten Wellstabilitäten beziehen sich auf diese Standardlösung (pH = 9), deren Wellstabilität auf 100 % gesetzt wurde.

Tabelle 1 zeigt, daß die Wellwirksamkeiten der in den erfindungsgemäßen Mitteln verwendeten 2-Mercaptoethylamine bei pH 7, 8 und 9 höher sind als bei Thiomilchsäure.

Tabelle 1

| **Versuchs-Nr.** | **Mercaptoethylamin** | **WSN** pH = 7 | **WSN** pH = 8 | **WSN** pH = 9 |
|---|---|---|---|---|
| 1 | 2,6-Dimethy-4-(2'-mercaptoethyl)-morpholin aus 2,6-Dimethylmorpholin | 63 | 63 | 68 |
| 2 | 2,6-Dimethyl-1-(2'-mercaptoethyl)-piperidin aus 2,6-Dimethylpiperidin | 61 | 74 | 71 |
| 3 | N-Cyclohexyl-N-2-mercaptoethylamin aus Cyclohexylamin | 77 | 83 | 86 |
| 4 | N-(4-Methyl-1-piperazinyl)-mercaptoethyl-amin aus N-Methylpiperazin | 70 | 91 | 93 |
| 5 | N-(2'-Mercaptoethyl)-piperidin-4-carbonsäure-ethylester aus Piperidin-4-carbonsäure-ethylester | 74 | 65 | 59 |
| 6 | 2-(2-Furanylmethyl)amino-mercaptoethylamin aus Furfurylamin | 85 | 80 | 87 |
| 7 | 2-Ethyl-N-(2'-mercaptoethyl)-piperidin aus 2-Ethylpiperidin | 74 | 75 | 77 |
| 8 | N-(2-Mercaptoethyl)-ethylamino-2-hydroxy-ethanol aus Ethylhydroxyethyl-amin | 74 | 82 | 96 |
| 9 | N-(2'-Mercaptoethyl)-morpholin aus Morpholin | 60 | 93 | 95 |
| 10 | N-(2'-Mercaptoethyl)-pyrrolidin aus Pyrrolidin | 85 | 96 | 97 |
| 11 | N-(2'-Mercaptoethyl)-piperidin aus Piperidin | 83 | 91 | 92 |
| | Thiomilchsäure als Vergleich | 57 | 50 | 70 |

**Beispiel 4: Dauerverformungsmittel für gefärbtes Haar**

| | |
|---|---|
| 9,76 g | N-(2'-Mercaptoethyl)-piperidin-4-carbonsäureethylester |
| 2,00 g | Ammoniumhydrogencarbonat |
| 2,00 g | Isopropanol |
| 1,00 g | Isooctylphenol, oxethyliert mit 10 mol Ethylenoxid |
| 1,00 g | Poly(dimethyldiallylammoniumchlorid) |
| 0,40 g | Ammoniak (25%ige wässrige Lösung) zur pH-Einstellung |
| 0,30 g | Parfümöl |
| 0,10 g | Vinylpyrrolidon/Styrol-Mischpolymerisat (Antara® 430 der GAF Corp.; NewYork/USA) |
| 83.44 g | Wasser |
| 100,00 g | |

Der pH-Wert dieses Mittels liegt bei 7,0 bis 7,5.

Durch Farbbehandlungen vorgeschädigtes Haar wird mit einem Shampoo gewaschen, frottiert und auf Wickler mit einem Durchmesser von 8 mm gewickelt. Anschließend wird das vorstehend beschriebene Haarverformungsmittel gleichmäßig auf dem gewickelten Haar verteilt. Sodann wird das Haar mit einer Plastikhaube abgedeckt und 10 Minuten lang unter einer Trockenhaube bei einer Temperatur von 45°C erwärmt. Anschließend wird die Abdeckung entfernt, das Haar mit Wasser gespült und mit 100 g einer 3%-igen wäßrigen Hydrogenperoxidlösung oxidativ nachbehandelt.

Nach Entfernung der Wickler werden die Haare erneut mit Wasser gespült, zur Wasserwelle gelegt und sodann getrocknet.
Als Ergebnis dieser Behandlung wird eine gleichmäßige, elastische und dauerhafte Verformung der Haare erhalten.

**Beispiel 5: Dauerwellverformungsmittel für normales Haar**

| | |
|---|---|
| 16,95 g | 2,6-Dimethyl-1-(2'-mercaptoethyl)piperidin |
| 8,90 g | Ammoniak (25%-ige wäßrige Lösung) |
| 5,00 g | Ammoniumhydrogencarbonat |
| 4,00 g | Harnstoff |
| 2,40 g | Monoethanolamin |
| 1,50 g | Isooctylphenol, oxethyliert mit 10 mol Ethylenoxid |
| 0,50 g | Poly(dimethyldiallylammoniumchlorid) |
| 0,50 g | Parfümöl |
| 0,10 g | Vinylpyrrolidon/Styrol-Mischpolymerisat (Antara® 430 der GAF Corp.; NewYork/USA) |
| 60,15g | Wasser |
| 100,00 g | |

Der pH-Wert dieses Mittels beträgt 8,0 bis 8,5.

Normales, nicht vorgeschädigtes Haar wird gewaschen, mit einem Handtuch frottiert und auf Wickler mit einem Durchmesser von 6 mm gewickelt. Anschließend wird das Haar mit dem vorstehend beschriebenen Haarverformungsmittel gleichmäßig durchfeuchtet. Nach einer Einwirkungszeit von 15 Minuten wird das Haar mit Wasser gründlich gespült und sodann mit 80 g einer 3%-igen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause.

**Beispiel 6: Dauerwellverformungsmittel für normales Haar**

| | |
|---|---|
| 6,42 g | N-(2'-Mercaptoethyl)-pyrrolidin |
| 4,51 g | Thioglykolsäure |
| 8,90 g | Ammoniak (25%-ige wäßrige Lösung) zur pH-Einstellung |
| 5,00 g | Ammoniumhydrogencarbonat |
| 4,00 g | Harnstoff |
| 2,40 g | Monoethanolamin |
| 1,50 g | 1sooctylphenol, oxethyliert mit 10 mol Ethylenoxid |
| 0,50 g | Poly(dimethyldiallylammoniumchlorid) |
| 0,50 g | Parfümöl |
| 0,10 g | Vinylpyrrolidon/Styrol-Mischpolymerisat (Antara® 430 der GAF Corp.; NewYork/USA) |
| 66.17 g | Wasser |
| 100,00 g | |

Der pH dieses Mittels liegt bei 8,0 bis 8,5.

Normales, nicht vorgeschädigtes Haar wird gewaschen, mit einem Handtuch frottiert und auf Wickler mit einem Durchmesser von 6 mm gewickelt. Anschließend wird das Haar mit dem vorstehend beschriebenen Haarverformungsmittel gleichmäßig durchfeuchtet. Nach einer Einwirkungszeit von 15 - 25 Minuten wird das Haar mit Wasser gründlich gespült und sodann mit 80 g einer 3%-igen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause.

## Patentansprüche

1. Mittel zur dauerhaften Verformung von Haaren, **dadurch gekennzeichnet**, daß es als keratinreduzierenden Wirkstoff ein Mercaptoethylamin der Formel

$$HS - CH_2 - CH_2 - NR_1 R_2 \qquad (I),$$

in der

$R_1$ Wasserstoff, einen geradkettigen oder verzweigten Alkyl-, Hydroxyalkyl- oder Polyhydroxyalkylrest mit 1 bis 6 Kohlenstoffatomen bedeutet;

$R_2$ ein geradkettiger oder verzweigter Hydroxyalkyl- oder Polyhydroxyalkylrest mit 1 bis 6 Kohlenstoffatomen, Cyclohexyl oder Furfuryl ist; oder

$R_1$ und $R_2$ gemeinsam mit dem Stickstoffatom einen fünf- oder sechsgliedrigen Ring bilden, der noch ein anderes Heteroatom enthalten kann und mit einer Hydroxyl- oder Carboxylgruppe oder mit einem oder zwei 1 bis 4 Kohlenstoffatome umfassenden Alkyl-, Hydroxyalkyl-, Polyhydroxyalkyl- oder Alkoxycarbonylrest substituiert ist, oder dessen physiologisch verträgliches Salz enthält.

2. Mittel zur dauerhaften Verformung von Haaren gemäß Anspruch 1, **dadurch gekennzeichnet,** daß es ein Mercaptoethylamin der Formel

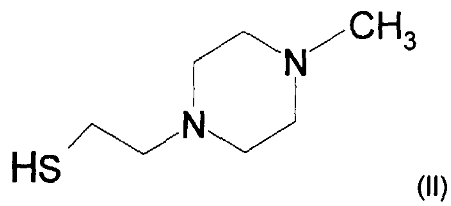

(II) oder

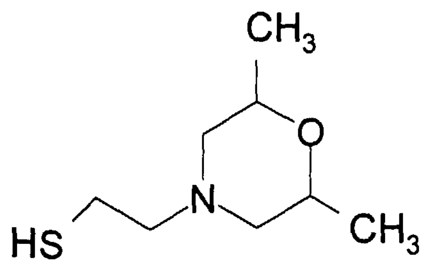

(III)

oder eines ihrer physiologisch verträglichen Salze enthält.

3. Mittel nach den Ansprüchen 1 oder 2, **dadurch gekennzeichnet,** daß es im gebrauchsfertigen Mittel das Mercaptoethylamin in einer Menge von 3 bis 28 Gewichtsprozent enthält.

4. Mittel nach einem der Ansprüche 1 bis 3, **dadurch gekennzeichnet,** daß der pH-Wert des gebrauchsfertigen Mittels 6,5 bis 9,5 beträgt.

5. Verfahren zur dauerhaften Verformung von Haaren, bei dem man das Haar bevor und/oder nachdem man es in der gewünschten Form festhält, mit einem Verformungsmittel behandelt, mit Wasser spült, oxidativ nachbehandelt, erneut mit Wasser spült, ggf. zur Wasserwelle legt und sodann trocknet, **dadurch gekennzeichnet,** daß man als Verformungsmittel ein Mittel nach den Ansprüchen 1 bis 4 verwendet.

6. Verfahren nach Anspruch 5, **dadurch gekennzeichnet,** daß man das Verformungsmittel 5 bis 30 Minuten auf das Haar einwirken läßt.

7. Verfahren nach Anspruch 6, **dadurch gekennzeichnet,** daß man das Verformungsmittel unter Anwendung von Wärme 5 bis 20 Minuten auf das Haar einwirken läßt.

8. Verfahren nach einem der Ansprüche 5 bis 7, **dadurch gekennzeichnet,** daß man das Verformungsmittel in einer Menge von 60 bis 120 g anwendet

Europäisches Patentamt

# EUROPÄISCHER RECHERCHENBERICHT

Nummer der Anmeldung
EP 98 10 2871

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | EP 0 465 342 A (OREAL) 8.Januar 1992<br>* Seite 11; Beispiel 3 *<br>--- | 1,3-8 | A61K7/09 |
| X | DATABASE WPI<br>Section Ch, Week 8221<br>Derwent Publications Ltd., London, GB;<br>Class D21, AN 82-42221E<br>XP002072079<br>& JP 57 062 217 A (KAO SOAP CO LTD)<br>* Zusammenfassung *<br>--- | 1,3,5 | |
| X | CHEMICAL ABSTRACTS, vol. 119, no. 8, 23.August 1993<br>Columbus, Ohio, US;<br>abstract no. 79822,<br>FUJIO, AKIRA ET AL: "Preparation of mercapto-modified lactams and odorless hair wave-setting compositions containing them"<br>XP002072078<br>* Zusammenfassung *<br>& JP 05 097 800 A (KAO CORP, JAPAN)<br>& RN=13839-15-1,<br>--- | 1,5 | RECHERCHIERTE SACHGEBIETE (Int.Cl.6)<br>A61K |
| X | EP 0 661 040 A (BRISTOL MYERS CO) 5.Juli 1995<br>* Ansprüche 1,2 *<br>--- | 1,3 | |
| D,Y | DE 195 03 131 A (WELLA AG) 8.August 1996<br>* das ganze Dokument *<br>--- | 1-8 | |
| Y | WO 93 06832 A (THOENE JESS G) 15.April 1993<br>* Anspruch 2 *<br>----- | 1-8 | |

Der vorliegende Recherchenbericht wurde für alle Patentansprüche erstellt

| Recherchenort | Abschlußdatum der Recherche | Prüfer |
|---|---|---|
| DEN HAAG | 20.Juli 1998 | Orviz Diaz, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)